# EUROPÄISCHE PATENTSCHRIFT

(11) **EP 2 039 670 B1**
(45) Veröffentlichungstag und Bekanntmachung des Hinweises auf die Patenterteilung: **10.03.2010**
(21) Anmeldenummer: 07116875.1
(22) Anmeldetag: 20.09.2007
(51) Int. Cl.: C07C 35/21, C07C 49/613, C07C 45/66, C11B 9/00, C11D 3/50

(54) **6-Methyl-4-(2',2',3'-trimethyl-3'-cyclopenten-1'-yl)-2-cyclohexen-1-ol als Sandelriechstoff**
6-methyl-4-(2',2',3'-trimethyl-3'-cyclopentene-1'-yl)-2-cyclohexene-1-ol as sandalwood aroma
6-méthyl-4-(2',2',3'-triméthyl-3'-cyclopentène-1'-yl)-2-cyclohexène-1-ol en tant que parfum de Santal

(43) Veröffentlichungstag der Anmeldung: 25.03.2009
(73) Patentinhaber: Symrise GmbH & Co. KG, 37603 Holzminden (DE)
(72) Erfinder: Brocke, Constanze, 64521 Groß-Gerau (DE); Eh, Marcus, 37603 Holzminden (DE); Oelkers, Egon, 37639 Bevern (DE)
(74) Vertreter: Eisenführ, Speiser & Partner

(56) Entgegenhaltungen:
- US-A- 4 188 310
- US-A- 5 189 013
- DATABASE CA [Online] CHEMICAL ABSTRACTS SERVICE, COLUMBUS, OHIO, US; LIU, DECHEN ET AL: "Synthesis of campholenyl cyclohexanol type sandalwood compounds" XP002469700 gefunden im STN Database accession no. 2000:372447 & HUAXUE SHIJI , 22(2), 100-102 CODEN: HUSHDR; ISSN: 0258-3283, 2000,

## Beschreibung

Die Erfindung betrifft eine Verbindung der Formel (VII) 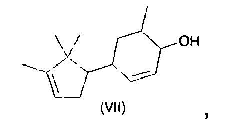 eine Riechstoff- oder Aromakomposition, umfassend eine solche Verbindung, sowie ein parfümiertes oder aromatisiertes Produkt, umfassend eine solche Verbindung. Die Erfindung betrifft ferner die Verwendung einer Verbindung der Formel (VII) als Riechstoff und/oder zur Verbesserung der Fixierung einer Riechstoff- oder Aromakomposition. Die Erfindung betrifft außerdem ein Verfahren zum Herstellen einer Verbindung der Formel (VII), ein Verfahren zum Herstellen eines Vorproduktes der Verbindung der Formel (VII) sowie das Vorprodukt selbst. Sie betrifft außerdem ein Verfahren zum Erzeugen, Verstärken oder Modifizieren eines Sandelholzgeruches in einer Mischung.

### Stand der Technik

In der Riechstoffindustrie besteht ein nachhaltiges Interesse an der Entwicklung von neuen Riechstoffen, um die Kreation von neuen Parfümölen für die alkoholische und für die funktionelle Parfümerie zu ermöglichen. Verbindungen mit holzigem Geruch sind hierbei unverzichtbare Komponenten in der Duftstoffindustrie. Eine besonders wertvolle Klasse dieser holzigen Riechstoffe sind Verbindungen mit Sandelholzgeruch. Strukturell zeichnen sich Verbindungen mit Sandelholzgeruch häufig durch ein 4-(2,2,3-Trimethyl-cyclopent-3-enyl)-butan-1-ol-Grundgerüst aus, wobei die Butan-1-ol-Seitenkette gesättigt oder einfach ungesättigt sowie ein- oder mehrfach methylsubstituiert sein kann. Einige Vertreter dieser Klasse von Riechstoffen sind 2-Methyl-4-(2',2',3'-trimethyl-3'-cyclopenten-1'-yl)-butan-1-ol (I) (Brahmanol^{®}, Symrise GmbH & Co. KG), 2-Ethyl-4-(2',2',3'-trimethyl-3'-cyclopenten-1'-yl)-2-buten-1-ol (II) (Sandranol^{®}, Symrise GmbH & Co. KG), 3-Methyl-5-(2',2',3'-trimethyl-3'-cyclopenten-1'-yl)-4-penten-2-ol (III) (Ebanol^{®}, Givaudan S.A.) und 3,3-Dimethyl-5-(2',2',3'-trimethyl-3'-cyclopenten-1'-yl)-4-penten-2-ol (IV) (Polysantol^{®}, Firmenich S.A.). 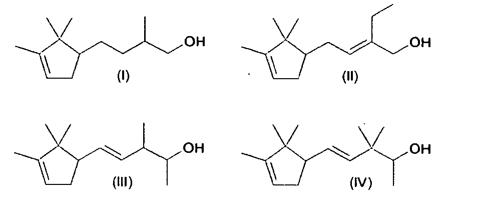 Diese Verbindungen (I) bis (IV) zeichnen sich durch einen starken Sandelholzgeruch aus, der (a) in der Stärke und (b) in weiteren Geruchsaspekten der einzelnen Verbindungen (I) - (IV) untereinander variiert.

Die US 5,189,013 offenbart Verbindungen, die unter die allgemeine Formel (V) fallen, das heißt Verbindungen, in denen anstelle einer Alkanol- bzw. Alkenol-Seitenkette (wie in den Verbindungen (I) bis (IV)) ein gesättigter oder ungesättigter Cyclohexanon- oder Cyclohexanol-Ring vorhanden ist. 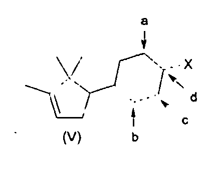 In der Formel (V) bedeutet die exocyclische gestrichelte Linie eine Einfachbindung (X ist dann OH) oder eine Doppelbindung (X ist dann O). Der sechsgliedrige Ring kann gemäß der allgemeinen Formel (V) in den Positionen a, b, c und d einen oder mehrere Methylsubstituenten besitzen. Offenbarte Verbindungen enthalten allerdings nur den Positionen c und d Substitutiomen. An Position a oder b substitiutierte Derivate werden nicht aufgeführt.

Die in der US 5,189,013 offenbarten Verbindungen weisen zumeist einen allenfalls schwachen Sandelholzgeruch auf. Lediglich hinsichtlich einer Verbindung, nämlich 1,2-Dimethyl-4-(2',2',3'-trimethyl-3'-cyclopenten-1'-yl)-2-cyclohexen-1-ol, wird ausgeführt, dass diese einen Geruch besitze, dessen Charakter dem des natürlichen Sandelholzes ähnlich sei. Die genannte Verbindung ist eine Verbindung der Formel (V), mit X gleich OH und in welcher der sechsgliedrige Ring eine Doppelbindung in Position 2 besitzt, das heißt zwischen den Pfeilen c und b (entspricht Verbindung (VIIIa), vgl. Tabelle 1). Darüber hinaus offenbart die US 5,189,013, dass keine der der letztgenannten Verbindung strukturell ähnlichen, offenbarten Verbindungen Geruchseigenschaften besitzt, die denen bekannter Verbindungen überlegen seien.

Die Publikationen Huaxue Shiji 2000, 22(2), 100-102 und Jingxi Huagong 1999, 16 (Zengkan, Proceedings for '99 China's Symposium on Technology Development and Application of Perfume and Essence), 294-297 beschreiben eine durch Reduktion von 4-(2',2',3'-Trimethyl-3'-cyclopenten-1'-yl)-2-cyclohexen-1-on erhaltene Mischung (VI) aus 4-(2',2',3'-Trimethyl-3'-cyclopenten-1'-yl)-2-cyclohexen-1-ol (VIa, ungesättigter Cyclohexanol-Ring, gestrichelte Linie entspricht Doppelbindung) und 4-(2',2',3'-Trimethyl-3'-cyclopenten-1'-yl)-cyclohexan-1-ol (VIb, gesättigter Cyclohexanol-Ring, gestrichelte Linie entspricht Einfachbindung), welche ein wertvolles, Sandelholz-artiges Aroma aufweist. 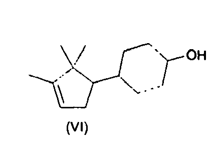 Aufgabe der vorliegenden Erfindung war es, einen neuen Sandelholz-Riechstoff anzugeben, der einen niedrigen geruchlichen Schwellenwert besitzt und hinsichtlich einiger oder der Summe seiner Sekundäreigenschaften den aus dem Stand der Technik bekannten Sandelholz-Riechstoffen überlegen ist. Erwünschte Sekundäreigenschaften in diesem Zusammenhang waren insbesondere eine hohe (Eigen-)Haftung, ein hoher Impact, eine hohe Substantivität, die Eigenschaft als Fixateur zu wirken sowie die Fähigkeit einen guten Blooming-Effekt zu bewirken.

Erfindungsgemäß wird diese Aufgabe gelöst durch eine Verbindung der Formel (VII).

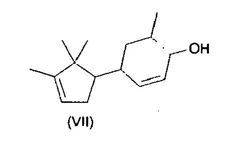

Die erfindungsgemäße Verbindung (VII) kann dabei bevorzugt in Form
(a) eines reinen optisch aktiven Enantiomers,
(b) einer racemischen Mischung der Enantiomere eines, zweier, dreier oder aller diastereomeren Enantiomerenpaare, oder
(c) einer optisch aktiven Mischung verschiedener Enantiomere vorliegen.

Die Verbindung der Formel (VII) besitzt vier chirale Zentren, so dass acht diastereomere Enantiomerenpaare existieren. Bei diesen acht diastereomeren Enantiomerenpaaren handelt es sich um folgende Verbindungen:
(1'R, 1 R, 4R, 6R)-6-Methyl-4-(2',2',3'-trimethyl-3'-cyclopenten-1'-yl)-2-cyclohexen-1-ol
(1'S, 1 S, 4S, 6S)-6-Methyl-4-(2',2',3'-trimethyl-3'-cyclopenten-1'-yl)-2-cyclohexen-1-ol
(1'R, 1S, 4R, 6R)-6-Methyl-4-(2',2',3'-trimethyl-3'-cyclopenten-1'-yl)-2-cyclohexen-1-ol
(1'S, 1R, 4S, 6S)-6-Methyl-4-(2',2',3'-trimethyl-3'-cyclopenten-1'-yl)-2-cyclohexen-1-ol
(1'R, 1R, 4S, 6R)-6-Methyl-4-(2',2',3'-trimethyl-3'-cyclopenten-1'-yl)-2-cyclohexen-1-ol
(1'S, 1S, 4R, 6S)-6-Methyl-4-(2',2',3'-trimethyl-3'-cyclopenten-1'-yl)-2-cyclohexen-1-ol
(1'R, 1R, 4R, 6S)-6-Methyl-4-(2',2',3'-trimethyl-3'-cyclopenten-1'-yl)-2-cyclohexen-1-ol
(1'S, 1S, 4S, 6R)-6-Methyl-4-(2',2',3'-trimethyl-3'-cyclopenten-1'-yl)-2-cyclohexen-1-ol
(1'R, 1S, 4S, 6R)-6-Methyl-4-(2',2',3'-trimethyl-3'-cyclopenten-1'-yl)-2-cyclohexen-1-ol
(1'S, 1R, 4R, 6S)-6-Methyl-4-(2',2',3'-trimethyl-3'-cyclopenten-1'-yl)-2-cyclohexen-1-ol
(1'R, 1S, 4R, 6S)-6-Methyl-4-(2',2',3'-trimethyl-3'-cyclopenten-1'-yl)-2-cyclohexen-1-ol
(1'S, 1R, 4S, 6R)-6-Methyl-4-(2',2',3'-trimethyl-3'-cyclopenten-1'-yl)-2-cyclohexen-1-ol
(1'R, 1R, 4S, 6S)-6-Methyl-4-(2',2',3'-trimethyl-3'-cyclopenten-1'-yl)-2-cyclohexen-1-ol
(1'S, 1S, 4R, 6R)-6-Methyl-4-(2',2',3'-trimethyl-3'-cyclopenten-1'-yl)-2-cyclohexen-1-ol
(1'R, 1S, 4S, 6S)-6-Methyl-4-(2',2',3'-trimethyl-3'-cyclopenten-1'-yl)-2-cyclohexen-1-ol
(1'S, 1R, 4R, 6R)-6-Methyl-4-(2',2',3'-trimethyl-3'-cyclopenten-1'-yl)-2-cyclohexen-1-ol.

Geruchlich besonders wertvoll sind Mischungen der Diastereomere der erfindungsgemäßen Verbindung, deren Zusammensetzung im Rahmen einer Gaschromatographiemessung Werte ergeben, die den in Tabelle 1 angegebenen Massenanteilen entsprechen oder Werte ergeben, die zwischen den jeweils angegebenen relativen minimalen und maximalen Massenanteilen liegen (GC-Säule: Zebron ZB-1 ms F&F 20 m L x 0.18 mm ID x 0.18 um df, Füllmaterial: 100% Dimethylpolysiloxane, Hersteller: Fa. Phenomenex, Programm: 60-12-300°C); angegeben sind die relativen Massenanteile der Peaks, bezogen auf den (kleinsten) Peak mit dem Retentionsindex 1632, sortiert nach Retentionsindices (vgl. Beispiel 1):

**Tabelle 1**

| Retentionsindex | Relativer Massenanteil |
|---|---|
| 1628 | 1.3-2.0 |
| 1632 | 1.0 |
| 1638 | 3.5-10.0 |
| 1644 | 2.8-8.0 |
| 1651 | 1.0-2.0 |

Die erfindungsgemäße Verbindung der Formel (VII) besitzt einen parfümistisch interessanten Sandelholzgeruch und zeichnet sich überdies durch erwünschte Sekundäreigenschaften aus: Insbesondere besitzt sie eine hohe (Eigen-)Haftung, einen niedrigen geruchlichen Schwellenwert, einen hohen Impact (Geruchsstärke), eine hohe Substantivität, sehr gute Fixateureigenschaften und einen ausgeprägten Blooming-Effekt. Dies ist insbesondere vor dem Hintergrund der in der US 5,189,013 gemachten Aussagen zu strukturähnlichen Verbindungen überraschend (vgl. oben).

Die (Eigen-)Haftung oder auch Aufziehvermögen genannt bezeichnet das Vermögen einer Verbindung, auf einem Substrat anzuhaften. Die Substantivität bezeichnet die Fähigkeit, aus einer meist wässrigen Phase heraus auf ein Substrat aufzuziehen bzw. auch nach einem Wasch- oder Spülvorgang auf einem Substrat zu verbleiben. Dieser Effekt zeigt sich insbesondere auf Substraten wie Haut, Haar und textilen Fasern (z. B. Baumwolle, Wolle, Leinen, synthetischen Fasern). Die Eigenschaft, als "Fixateur" wirken zu können (Fixateureigenschaft) bedeutet, dass die entsprechende Verbindung die Haftfestigkeit von anderen Riechstoffen erwirkt. Dies kann z. B. durch Dampfdruckerniedrigung oder geruchliche Verstärkung (z. B. Absenkung des Schwellenwertes erfolgen). Der Blooming-Effekt ist der über einer tensidhaltigen wässrigen Lösung wahrgenommene Geruch.

Die erfindungsgemäße Verbindung der Formel (VII) zeigt sowohl im Angeruch als auch im Nachgeruch eine starke, strahlende, schöne natürliche Sandelholznote mit etwas süßlich-milchigen und moschushaften Aspekten. Sie zeichnet sich insbesondere durch ihr komplexes Sandelholz-Geruchsbild aus, welches den Geruch des natürlich vorkommenden Sandelholzöls in seinem Facettenreichtum nahezu imitiert. Die erfindungsgemäße Verbindung besitzt demnach eine sensorisch sehr wertvolle intensive, natürliche Sandelholznote, gepaart mit einer überraschend hohen Haftung. In Parfümöl-Kompositionen (vgl. Beispiel 2) zeigt sie während des gesamten Duftablaufs eine positive Wirkung, indem sie den Sandelcharakter der Komposition deutlich verstärkt und eine gute Haftung bietet.

Dabei unterscheidet sich die erfindungsgemäße Verbindung der Formel (VII) überraschenderweise deutlich von den in Huaxue Shiji 2000, 22(2), 100-102 bzw. Jingxi Huagong 1999, 16, 294-297 beschriebenen Verbindungen (VIa) und (Vlb) (vgl. Tabelle 2). Bei der Verbindung (VIa) handelt es sich um einen sehr sauberen, aber dadurch wesentlich schlanker wirkenden Sandelriechstoff, der nicht so natürlich wirkt wie die erfindungsgemäße Verbindung der Formel (VII). Die reine Verbindung (Vlb) zeigt sogar keinen Sandelgeruch.

Auch von den in US 5,189,013 beschriebenen Verbindungen unterscheidet sich die erfindungsgemäße Verbindung der Formel (VII) deutlich sowohl strukturell als auch geruchlich (vgl. Tabelle 1). Beispielsweise enthält die Verbindung (VIIIa) eine zusätzliche Methylgruppe und beinhaltet eine tertiäre Alkoholfunktion (im Gegensatz zu der in Formel (VII) vorliegenden sekundären Hydroxygruppe), zusätzlich zu der an anderer Position im Ring auftretenden Doppelbindung. Geruchlich zeigt die Verbindung (VIIIa) im Angeruch eine holzige Note, die jedoch eher in Richtung Zedernholz und Patchouli tendiert und nicht typisch sandelig ist. Der Nachgeruch dieser Verbindung ist deutlich schwächer als der Angeruch. Verbindung (IXa) wird ebenfalls durch eine tertiäre (im Gegensatz zu Verbindung (VII) sekundäre) Alkoholfunktion charakterisiert. Hierbei handelt es sich um eine schwach holzigriechende Verbindung mit balsamischer, ambrahafter (und nicht sandeliger) Beinote, die im Angeruch stärker wirkt als im Nachgeruch, wodurch es dem Stoff an Ausdruck mangelt. Schließlich zeigt die Verbindung (Xa) im Unterschied zu der erfindungsgemäßen Verbindung der Formel (VII) keine typische Sandelnote, sondern nach einem schwachen Angeruch später eine süßlich-holzige, Cananga-artige Note. Bei dieser Verbindung handelt es sich um einen nur sehr schwachen Riechstoff.

**Tabelle 2**

| Nr. | Struktur | Geruchsbeschreibung |
|---|---|---|
| VIa | 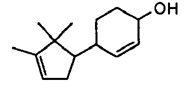 | Sandelholz-artige Note |
| Vlb | 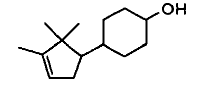 | süß, Moschus, animalisch |
| VIIIa | 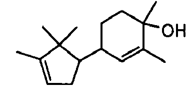 | kräftige, holzige Note mit klarem, sauberem Sandelholz-Charakter, der sich erst in der Basisnote entfaltet* |
| Vlllb | 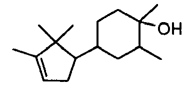 | holzig, phenolisch, Leder, schwach nach Sandelholz* |
| Vlllc | 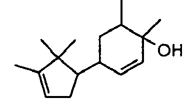 | Acetat, Holz, sehr schwache Sandelnote |
| IXa | 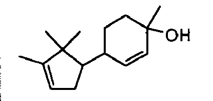 | schwach holzig, sehr schwach nach Sandelholz, fruchtig* |
| IXb | 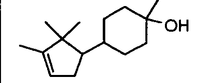 | schwach; schwach, blumig, dreckig* |
| Xa | 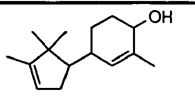 | schwach, schwach nach Sandelholz* |
| Xb | 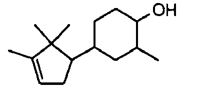 | schwach, blumig, holzig, Sandelholz-artig* |

| | | |
|---|---|---|
| *Geruchsbeschreibungen gemäß US 5,189,013. | | |

Es ist vorteilhaft, die erfindungsgemäße Verbindung zumindest mit einem weiteren Riech- oder einem Aromastoff zu kombinieren und so eine neue Riech- oder Aromastoffkomposition zu bilden. Auf diese Weise lassen sich interessante und natürliche neue und originelle Duftnoten kreieren. Riechstoffe, die zur Kombination vorteilhafterweise geeignet sind, finden sich z.B. in S. Arctander, Perfume and Flavor Materials, Vol. I und II, Montclair, N. J. 1969, Eigenverlag, oder H. Surburg, J. Panten, Common Fragrance and Flavor Materials, 5th Edition, Wiley-VCH, Weinheim 2006. Im einzelnen seien genannt:
**Extrakte aus natürlichen Rohstoffen** wie Etherische Öle, Concretes, Absolues, Resine, Resinoide, Balsame, Tinkturen wie z. B.
Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
**Einzel-Riechstoffe** aus der Gruppe der Kohlenwasserstoffe, wie z.B. 3-Caren; alpha-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)-und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; Isopulegol; alpha-Terpineol; Terpinenol-4; Menthan-8-ol; Menthan-1-ol; Menthan -7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether;1,1-Dimethoxycyclododecan;
   (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat;
Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

In Riech- oder Aromastoffkompositionen beträgt die eingesetzte Menge der erfindungsgemäßen Verbindung vorzugsweise 0,0001 bis 90 Gew.-%, vorzugsweise 0,01 bis 70 Gew.-% und besonders bevorzugt 0,1 bis 50 Gew.-%, bezogen auf die Gesamtmenge der Riech- oder Aromastoffkomposition.

Riech- oder Aromastoffkompositionen, die die erfindungsgemäße Verbindung enthalten, können in flüssiger Form, unverdünnt oder mit einem Lösungsmittel verdünnt zur Parfümierung oder Aromatisierung eingesetzt werden. Geeignete Lösungsmittel hierfür sind z.B. Ethanol, Isopropanol, Diethylenglycolmonoethyleter, Glycerin, Propylenglycol, 1,2-Butylenglycol, Dipropylenglycol, Diethylphtalat, Triethylcitrat, Isopropylmyristat, Triacetin, Pflanzenöle usw.

Zutaten, mit denen die erfindungsgemäße Verbindung kombiniert werden kann, sind beispielsweise:

Konservierungsmittel, Abrasiva, Antiakne-Mittel, Mittel gegen Hautalterung, anitbakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Slilcone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Des weiteren können erfindungsgemäße Riech- oder Aromastoffkompositionen, die die erfindungsgemäße Verbindung enthalten, an einem Trägerstoff adsorbiert sein, der sowohl für eine feine Verteilung der Riech- oder Aromastoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker, Dextrine (z.B. Maltodextrin) oder Kunststoffe wie PVC, Polyvinylacetate oder Polyurethane sein. Die Kombination aus erfindungsgemäßer Komposition und Trägerstoff stellt einen beispielhaften erfindungsgemäßen Artikel dar.

Riech- oder Aromastoffkompositionen, die die erfindungsgemäße Verbindung enthalten, können auch mikroverkapselt, sprühgetrocknet, als Einschluss-Komplexe oder als Extrusions-Produkte (d. h. erfindungsgemäße Produkte) vorliegen und in dieser Form z. B. einem zu parfümierenden oder zu aromatisierenden Produkt hinzugefügt werden.

Gegebenenfalls können die Eigenschaften der derart modifizierten Kompositionen durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden. Die resultierenden Produkte stellen wiederum erfindungsgemäße Produkte dar.

Die Mikroverkapselung der erfindungsgemäßen Riech- oder Aromastoffkompositionen zu erfindungsgemäßen Produkte kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Riech- oder Aromastoffkompositionen können beispielsweise durch Sprühtrocknung einer die Riech- oder Aromastoffkomposition enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von der Riech- oder Aromastoffkomposition und Cyclodextrinen oder Harnstoffderivaten in ein geeignetes Lösungsmittel, z.B. Wasser, hergestellt werden. Extrusions-

Produkte können durch Verschmelzen der Riech- oder Aromastoffkompositionen mit einem geeigneten wachsartigen Stoff und durch Extrusion mit nachfolgender Erstarrung, ggf. in einem geeigneten Lösungsmittel, z.B. Isopropanol, erhalten werden.

Die erfindungsgemäße Verbindung und Riechstoffkompositionen, die die erfindungsgemäße Verbindung enthalten, können in konzentrierter Form, in Lösungen oder in oben beschriebener modifizierter Form verwendet werden für die Herstellung von erfindungsgemäßen parfümierten Produkten wie z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Textilerfrischern, Bügelhilfen, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und - lotionen, Sonnenschutzcremes-und -lotionen, After-sun-cremes und -lotionen, Handcremes und -lotionen, Fußcremes und -lotionen, Enthaarungscremes und - lotionen, After-shave-Cremes und -lotionen, Bräunungscremes und -lotionen, Haarpflegeprodukten wie z.B. Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien und Treibstoffen.

Die erfindungsgemäße Verbindung kann in aromatisierte oder zu aromatisierende Produkte eingearbeitet werden, insbesondere der Ernährung, der Mundpflege oder dem Genuss dienende Zubereitungen. Solche Produkte umfassen regelmäßig Bestandteile, die andere als auf die Aroma- oder Duftentwicklung bezogene Funktionen haben (z. B. Farbstoffe) und/oder sind ein bereits fertiges handelbares Produkt (z. B. Parfümöl).

Der Ernährung oder dem Genuss dienende Zubereitungen sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nichtalkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Nach Einarbeiten der erfindungsgemäßen Verbindung sind diese Zubereitungen erfindungsgemäße Zubereitungen (als Beispiel erfindungsgemäßer Produkte).

Erfindungsgemäße Zubereitungen können z. B. als Halbfertigware oder als Würzmischung vorliegen.

Erfindungsgemäße Zubereitungen können insbesondere als Halbfertigware zur Herstellung weiterer der Ernährung oder dem Genuss dienenden Zubereitungen dienen, insbesondere in sprühgetrockneter Form. Erfindungsgemäße Zubereitungen können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Der Mundpflege dienende erfindungsgemäße Zubereitungen sind insbesondere Mund- und/oder Zahnpflegemittel wie Zahnpasten, Zahngele, Zahnpulver, Mundwässer, Kaugummis und andere Mundpflegemittel.

Weitere übliche Wirk-, Grund-, Hilfs- und Zusatzstoffe für der Ernährung, der Mundpflege oder dem Genuss dienende erfindungsgemäße Zubereitungen können in Mengen von 5 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, aufweisen.

Die erfindungsgemäßen Zubereitungen (als Beispiele erfindungsgemäßer Produkte), enthaltend die erfindungsgemäße Verbindung, werden gemäß einer bevorzugten Ausgestaltung hergestellt, indem die erfindungsgemäße Verbindung als Substanz, als Lösung (z.B. in Ethanol, Wasser oder 1,2-Propylenglycol) oder in Form eines Gemisches mit einem festen oder flüssigen Trägerstoff (z.B. Maltodextrin, Stärke, Silicagel), sonstigen Aromen oder Aromastoffen und gegebenfalls weiteren Hilfsmitteln und/oder Stabilisatoren (z.B. natürliche oder künstliche Polysaccharide und/oder Pflanzengummen wie modifizierte Stärken oder Gummi Arabicum) in eine der Ernährung, der Mundpflege oder dem Genuss dienende Basis-Zubereitung eingearbeitet werden. Vorteilhafterweise können als Lösung und/oder Suspension oder Emulsion vorliegende erfindungsgemäße Zubereitungen auch durch Sprühtrocknung in eine feste erfindungsgemäße Zubereitung (Halbfertigware) überführt werden.

Die erfindungsgemäßen sprühgetrockneten festen Zubereitungen (als Beispiel erfindungsgemäßer Produkte) sind als Halbfertigwaren besonders gut zur Herstellung von weiteren erfindungsgemäßen Zubereitungen geeignet. In den erfindungsgemäßen sprühgetrockneten festen Zubereitungen sind vorzugsweise 50 bis 95 % Gew.-% Trägerstoffe, insbesondere Maltodextrin und/oder Stärke, 5 bis 40 % Hilfsstoffe, bevorzugt natürliche oder künstliche Polysaccharide und/oder Pflanzengummen wie modifizierte Stärken oder Gummi Arabicum enthalten.

Gemäß einer weiteren bevorzugten Ausführungsform werden zur Herstellung erfindungsgemäßer Zubereitungen die erfindungsgemäße Verbindung und gegebenenfalls andere Bestandteile der erfindungsgemäßen Zubereitung zunächst in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatine oder sonstigen Naturprodukten (z.B. Schellack) eingearbeitet. Dabei können je nach Matrix die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und gegebenenfalls eine geeignete Kombination der vorgenannten Verfahren erhalten werden. In einem weiteren bevorzugten Herstellungsverfahren für eine erfindungsgemäße Zubereitung wird die erfindungsgemäße Verbindung zunächst mit einem oder mehreren geeigneten Komplexbildnern, beispielsweise mit Cyclodextrinen oder Cyclodextrinderivaten, bevorzugt α- oder β-Cyclodextrin, komplexiert und in dieser komplexierten Form eingesetzt.

Besonders bevorzugt ist eine erfindungsgemäße Zubereitung, bei der die Matrix so gewählt ist, dass die erfindungsgemäße Verbindung verzögert von der Matrix freigegeben wird, so dass eine langanhaltende Wirkung erzielt wird. Besonders bevorzugt ist insoweit eine Fett-, Wachs-, Polysaccharid- oder Proteinmatrix.

Als weitere Bestandteile für erfindungsgemäße, der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nichtproteinogene Aminosäuren und verwandte Verbindungen (z.B. gamma-Aminobuttersäure, Taurin), Peptide (z.B. Glutahthion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Zahnpflegemittel (als Basis für der Mundpflege dienende Zubereitungen), die die erfindungsgemäße Verbindung enthalten, umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für der Mundpflege dienende Zubereitungen), welche die erfindungsgemäße Verbindung enthalten, umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkoholen, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Bevorzugt können die erfindungsgemäßen Zubereitungen auch neben der erfindungsgemäß einzusetzenden Verbindung eine (zusätzliche) Aromakomposition enthalten, um den Geschmack und/oder Geruch der Zubereitung abzurunden und zu verfeinern. Geeignete (zusätzliche) Aromakompositionen enthalten z.B. synthetische, natürliche oder naturidentische Aroma-, Riech- und Geschmacksstoffe sowie geeignete Hilfs- und Trägerstoffe.

Selbstverständlich ist es bevorzugt - wenn auch nicht zwingend notwendig -, dass erfindungsgemäße Produkte bzw. Riechstoff- oder Aromastoffkompositionen eine als Sandelholzgeruchsnote olfaktorisch wahrnehmbare Menge einer erfindungsgemäßen Verbindung umfassen. In diesem Fall kommt (auch) die positive Primäreigenschaft der erfindungsgemäßen Verbindung deutlich zum Tragen. Für einige Anwendungen kann es aber vorteilhaft sein, nur die positiven Sekundäreigenschaften der erfindungsgemäßen Verbindung der Formel (VII) auszunutzen.

Bestandteil der Erfindung ist auch ein erfindungsgemäßes Produkt (umfassend eine erfindungsgemäße Verbindung der Formel (VII)), umfassend einen Träger oder ein Substrat, der bzw. das in direktem Kontakt mit der erfindungsgemäßen Verbindung der Formel (VII) bzw. mit der Riechstoff- oder Aromakomposition steht. Geeignete Träger sind weiter oben aufgeführt.

Bestandteil der Erfindung ist auch die Verwendung einer erfindungsgemäßen Verbindung (einer Verbindung der Formel (VII)) als Riechstoff.

Durch die Verwendung der erfindungsgemäßen Verbindung der Formel (VII) lassen sich bereits in geringer Dosierung in den resultierenden Parfümkompositionen (Riechstoffmischungen) Sandelholznoten erreichen, die sehr deutlich an Sandelholzöl erinnern, wobei der geruchliche Gesamteindruck auffallend harmonisiert, die Ausstrahlung wahrnehmbar erhöht und die Fixierung, d.h. das Haftvermögen der Parfümkomposition, deutlich verstärkt sind (vgl. Beispiel 2).

Im Vergleich zu kommerziell erhältlichen Sandelriechstoffen wie Ebanol^{®} (III) oder Polysantol^{®} (IV) zeigt die Verbindung (VII) einen ähnlichen Schwellenwert, Impact und Blooming-Effekt (das ist der über einer tensidhaltigen wässrigen Lösung wahrgenommene Geruch) sowie eine ähnliche Intensität. Die Verbindung (VII) zeichnet sich jedoch insbesondere im Vergleich zu Polysantol^{®} (IV) durch eine wesentlich höhere (Eigen)Haftung aus. Die erfindungsgemäße Verbindung der Formel (VII) lässt sich daher insbesondere als Fixateur in Parfümkompositionen einsetzen. Daneben zeigt die erfindungsgemäße Verbindung der Formel (VII) eine bemerkenswerte Substantivität.

Dementsprechend ist aufgrund der erwünschten sekundären Eigenschaften der erfindungsgemäßen Verbindung der Formel (VII) auch Bestandteil der Erfindung eine Verwendung der erfindungsgemäßen Verbindung zur Verbesserung der Fixierung einer Riechstoff- oder Aromastoffkomposition, wobei eine gleichzeitige Verwendung der erfindungsgemäßen Verbindung auch als Riechstoff bevorzugt ist.

Wie im Folgenden noch näher erläutert wird (vgl. den Syntheseweg gemäß Schema 1 sowie Beispiel 1), lässt sich die erfindungsgemäße Verbindung der Formel (VII) in besonders wirtschaftlicher Weise synthetisieren. 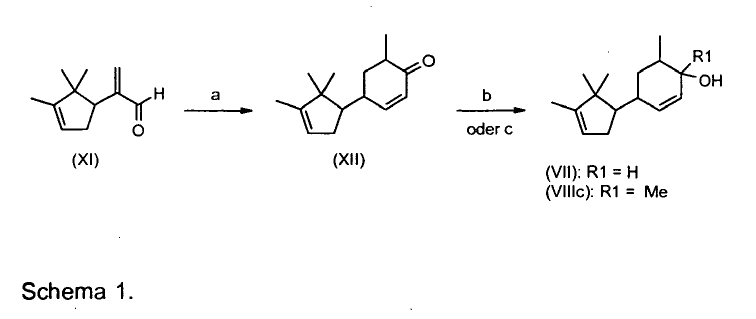
a) 2-Methylacetessigsäureethylester, kat. KOtBu, tBuOH; b) LiAlH₄, Et₂O; c) MeMgCl, THF.

Wie aus dem Schema 1 hervorgeht, führt die Synthese der erfindungsgemäßen Verbindung der Formel (VII) über ein Zwischenprodukt, nämlich eine Verbindung der Formel (XII) 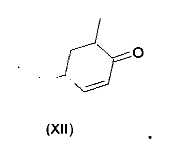 Dieses Zwischenprodukt ist neu und damit auch Bestandteil der Erfindung.

Dementsprechend ist ebenfalls Bestandteil der Erfindung ein Verfahren zur Herstellung einer Verbindung der Formel (XII) 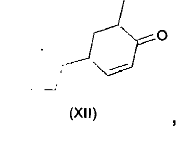 umfassend die Schritte
- Bereitstellen von α-Methylencampholenaldehyd der Formel (XI) 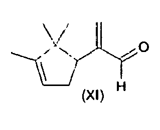
- Umsetzen des bereitgestellten α-Methylencampholenaldehyd der Formel (XI) mit 2-Methylacetessigsäureethylester in einer basenkatalysierten Cyclisierungs-Reaktion.

Mechanistisch umfasst das erfindungsgemäße Verfahren vermutlich eine Tandem-Michael Addition-Aldolreaktion und eine in situ-Lactonisierung und Decarboxylierung, wobei 2-Methylacetessigsäureethylester in Gegenwart von Kalium-tert-butanolat als Base umgesetzt wird (vergleiche J. Org. Chem. 1997, 62, 9323-9325).

Die Erfindung betrifft auch ein Verfahren zur Herstellung der erfindungsgemäßen Verbindung (VII) mit folgenden Schritten:
- Bereitstellen oder Herstellen einer Verbindung der Formel (XII), 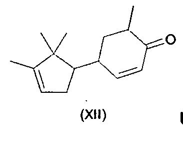 und
- Reduzieren der Verbindung (XII), so dass die erfindungsgemäße Verbindung (VII) gebildet wird.

In dem erfindungsgemäßen Verfahren zur Herstellung der Verbindung der Formel (VII) erfolgt die Reduktion der Verbindung vorzugsweise mit einem Reduktionsmittel wie Lithiumaluminiumhydrid.

Ausgehend von der Verbindung der Formel (XI) lässt sich in einem bevorzugten erfindungsgemäßen Verfahren insgesamt in zwei Schritten die Verbindung der Formel (VII) herstellen. Das oben gezeigte Schema 1 verdeutlicht die durchzuführenden Reaktionsschritte, wobei für die Verbindung der Formel (VII) R1 = H gilt.

Der in Schema 1 gezeigte Syntheseweg unterscheidet sich deutlich von dem in US 5,189,013 beschriebenen Herstellungsverfahren der Vergleichssubstanzen. Die Synthese der in US 5,189,013 beschriebenen Verbindungen erfolgt ausgehend von α-Campholenaldehyd, welcher in ein Enamin überführt und anschließend in einer Michael-Addition mit Alkylvinylketon umgesetzt wird. Es folgen die Cyclisierung zu den entsprechenden 2-Cyclohexenon-Derivaten durch Aldol-Kondensation und weitere Derivatisierungen zu den verschiedenen Alkoholen.

Bei dem in Schema 1 beschriebenen Herstellverfahren erfolgt hingegen im ersten Schritt die basenkatalysierte Cyclisierung von α-Methylencampholenaldehyd (XI) mit 2-Methylacetessigsäureethylester zu dem Cyclohexenon-Derivat (XII). Dieses lässt sich durch dem Fachmann bekannte weitere Reaktionsschritte in die Verbindungen der Formeln (VII) (R1 = H) und (Vlllc) (R1 = Me) umwandeln. Reduktion der Verbindung (XII) ergibt den erfindungsgemäßen sekundären Alkohol (VII), während eine Grignard-Reaktion mit Methylmagnesiumchlorid den tertiären Alkohol (VIIIc) liefert.

Die Erfindung betrifft auch ein Verfahren zum Erzeugen, Verstärken oder Modifizieren eines Sandelholzgeruches in einer Mischung, umfassend folgende Schritte:
- Bereitstellen einer erfindungsgemäßen Verbindung,
- Bereitstellen einer Komposition sonstiger Bestandteile und
- Vermischen der Komposition sonstiger Bestandteile mit einer Menge der erfindungsgemäßen Verbindung, die ausreicht, um
   (a) in der resultierenden Gesamtmischung einen Sandelholzgeruch zu erzeugen,
   (b) einen vorhandenen Sandelholzgeruch in der Komposition sonstiger Bestandteile zu verstärken oder
   (c) einen vorhandenen Sandelholzgeruch in der Komposition sonstiger Bestandteile zu modifizieren.

Die folgenden, nicht limitierenden Beispiele erläutern die Erfindung weiter.

Die Angaben zu den Retentionsindices (RI) in der Gaschromatographie (GC) der Beispiele 1.1 und 1.2 beziehen sich auf die folgende GC-Säule: Zebron ZB-1ms F&F 20 m L x 0.18 mm ID x 0.18 µm df, Füllmaterial: 100% Dimethylpolysiloxane, Hersteller: Fa. Phenomenex, Programm: 60-12-300°C.

### Beispiel 1: Synthese von 6-Methyl-4-(2',2',3'-trimethyl-3'-cyclopenten-1'-yl)-2-cyclohexen-1-ol (VII)

### Beispiel 1.1: Synthese von 6-Methyl-4-(2',2',3'-trimethyl-3'-cyclopenten-1'-yl)-2-cyclohexen-1-on (XII)

Eine Lösung aus 2-Methylacetessigsäureethylester (3.75 g, 26 mmol) und α-Methylencampholenaldehyd (XI) (4.27 g, 26 mmol) in 25 mL tert-Butanol wird bei 15°C unter Rühren portionsweise mit Kalium-tert-butanolat (1.46 g, 13 mmol) versetzt. Anschließend wird das Reaktionsgemisch drei Stunden am Rückfluss erhitzt. Zur Aufarbeitung wird die Reaktionslösung auf Raumtemperatur abgekühlt, mit verdünnter Salzsäure (15 mL) versetzt und mit Diethylether verdünnt. Nach Phasentrennung wird die organische Phase mit verdünnter Natronlauge, mit gesättigter Natriumchlorid-Lösung und mit dest. Wasser neutral gewaschen, über Natriumsulfat getrocknet und im Vakuum eingeengt. Säulenchromatographische Reinigung des Rohproduktes an Silicagel (Laufmittel: Cyclohexan/Essigsäureethylester 20/1) ergibt 6-Methyl-4-(2',2',3'-trimethyl-3'-cyclopenten-1'-yl)-2-cyclohexen-1-on (XII) in Form eines farblosen Öls (3.0 g, 53%).

Die vom Produkt gemessenen ¹H-NMR- und ¹³C-NMR-Spektren entsprechen einem Stereoisomerengemisch der Verbindung 6-Methyl-4-(2',2',3'-trimethyl-3'-cyclopenten-1'-yl)-2-cyclohexen-1-on (XII).

### GC-Peak 1 (RI = 1671):

MS (EI): m/z = 41 (37), 53 (21), 55 (33), 67 (68), 77 (26), 79 (36), 81 (48), 91 (29), 93 (35), 95 (84), 109 (100), 110 (35), 147 (43), 203 (27), 218 (15, M^{·+}).

### GC-Peak 2 (RI = 1676):

MS (EI): m/z = 41 (48), 43 (20), 53 (28), 55 (45), 67 (78), 77 (33), 79 (49), 81 (60), 91 (43), 93 (64), 95 (96), 105 (28), 106 (42), 107 (27), 108 (100), 109 (100), 110 (25), 119 (22), 133 (23), 147 (58), 161 (29), 175 (72), 203 (26), 218 (27, M^{·+}).

### GC-Peak 3 (RI = 1679):

MS (EI): m/z = 41 (28), 55 (27), 67 (52), 77 (20), 79 (29), 80 (27), 81 (42), 91 (24), 93 (27), 95 (76), 108 (22), 109 (100), 110 (27), 147 (43), 203 (48), 218 (12, M^{·+}).

Geruchsbeschreibung von 6-Methyl-4-(2',2',3'-trimethyl-3'-cyclopenten-1'-yl)-2-cyclohexen-1-on (XII): holzige Note in Richtung Zedernholz.

### Beispiel 1.2: Synthese von 6-Methyl-4-(2',2',3'-trimethyl-3'-cyclopenten-1'-yl)-2-cyclohexen-1-ol (VII)

Zu einer Suspension aus Lithiumaluminiumhydrid (133 mg, 3.5 mmol) in 6 mL Diethylether wird bei 15°C 6-Methyl-4-(2',2',3'-trimethyl-3'-cyclopenten-1'-yl)-2-cyclohexen-1-on (XII) (1.53 g, 7 mmol), gelöst in 3 mL Diethylether, tropfenweise zugegeben. Das Reaktionsgemisch wird 90 Minuten gerührt. Zur Aufarbeitung wird das überschüssige Lithiumaluminiumhydrid durch Zugabe von wenigen Tropfen dest. Wasser hydrolysiert, und der entstandene Niederschlag wird abfiltriert. Die organische Phase wird über Natriumsulfat getrocknet und im Vakuum eingeengt. Säulenchromatographische Reinigung des Rohproduktes an Silicagel (Laufmittel: Cyclohexan/Essigsäureethylester 3/1) ergibt 6-Methyl-4-(2',2',3'-trimethyl-3'-cyclopenten-1'-yl)-2-cyclohexen-1-ol (VII) in Form eines farblosen Öls (1.26 g, 81%).

Die vom Produkt gemessenen ¹H-NMR- und ¹³C-NMR-Spektren entsprechen einem Stereoisomerengemisch der Verbindung 6-Methyl-4-(2',2',3'-trimethyl-3'-cyclopenten-1'-yl)-2-cyclohexen-1-ol (VII).

GC: RI = 1628 (8%), 1632 (5%), 1638 (43%), 1644 (35%), 1651 (8%).

### GC-Peak 1 (RI = 1628):

MS (EI): m/z = 41 (16), 43 (15), 55 (14), 67 (33), 77 (11), 79 (14), 81 (15), 91 (12), 93 (33), 95 (29), 108 (100), 109 (35), 110 (13), 220 (0.1, M^{·+}).

### GC-Peak 2 (RI = 1632):

MS (EI): m/z = 41 (16), 43 (13), 55 (13), 67 (27), 77 (11), 79 (13), 81 (13), 91 (13), 93 (32), 95 (18), 108 (100), 109 (29), 220 (0.1, M^{·+}).

### GC-Peak 3 (RI = 1638):

MS (EI): m/z = 41 (25), 43 (27), 53 (11), 55 (29), 67 (54), 77 (19), 79 (23), 81 (30), 91 (24), 93 (50), 94 (11), 95 (69), 107 (20), 108 (55), 109 (59), 110 (20), 111 (11), 119 (11), 121 (13), 145 (11), 187 (13), 205 (100), 206 (16), 220 (4, M^{·+}).

### GC-Peak 4 (RI = 1644):

MS (EI): m/z = 29 (10), 41 (29), 43 (34), 53 (12), 55 (33), 67 (70), 77 (21), 79 (26), 81 (35), 91 (25), 93 (46), 94 (13), 95 (84), 96 (13), 105 (11), 107 (18), 108 (71), 109 (100), 110 (53), 111 (15), 119 (12), 121 (14), 145 (11), 159 (12), 187 (17), 220 (7, M^{·+}).

### GC-Peak 5 (RI = 1651):

MS (EI): m/z = 29 (11), 39 (11), 41 (35), 43 (35), 53 (14), 55 (34), 67 (77), 69 (11), 77 (24), 79 (28), 81 (37), 91 (27), 93 (46), 94 (13), 95 (84), 96 (11), 105 (11), 107 (19), 108 (88), 109 (100), 110 (58), 111 (17), 119 (11), 121 (12), 187 (11), 205 (36), 220 (2, M^{·+}).

Geruchsbeschreibung von 6-Methyl-4-(2',2',3'-trimethyl-3'-cyclopenten-1'-yl)-2-cyclohexen-1-01 (VII): starke, strahlende, schöne natürliche Sandelholznote mit süßlich-milchigen und moschushaften Aspekten

### Beispiel 2: Parfümöl-Komposition

Das nachfolgend angegebene Parfümöl kann zur Parfümierung diverser kosmetischer Produkte verwendet werden.

### Zusammensetzung:

| Ingredienzien | Gewichtsteile |
|---|---|
| Linalylacetat | 40,0 |
| Oxania Type Base, 10%ig in DPG | 1,0 |
| Lilial^{®1} (2-Methyl-3-(4-tert-butylphenyl)propanal) | 5,0 |
| Helional^{®2} (alpha-Methyl-1,3-benzodioxol-5-propanal) | 2,0 |
| Florosa^{®1} (4-Methyl-2-(2-methylpropyl)tetrahydro-2H-4-pyranol) | 5,5 |
| Hydroxycitronellal | 17,0 |
| Mayol^{®3} (cis-4-(1-Methylethyl)cyclohexanmethanol) | 5,5 |
| Linalool | 20,0 |
| Citronellol, 10%ig in DPG | 3,0 |
| Geraniol, 10%ig in DPG | 2,0 |
| Geranylacetat, 10%ig in DPG | 1,0 |
| Nerylacetat, 10%ig in DPG | 1,0 |
| Benzylacetat | 7,5 |
| Hedion^{®3} (Methyldihydrojasmonat) | 84,0 |
| Hexylzimtaldehyd alpha | 6,5 |
| Iraldein gamma | 11,0 |
| Iron alpha, 10%ig in DPG | 1,0 |
| Vanillin | 7,0 |
| Agrumex HC^{®4}, 10%ig in DPG (2-tert-Butylcyclohexylacetat) | 1,0 |
| Herbaflorat^{®4}, 10%ig in DPG (4,7-Methano-3a,4,5,6,7,7a- hexahydro-5-indenylacetat und/oder 4,7-Methano-3a,4,5,6,7,7a-hexahydro-6-indenylacetat) | 0,5 |
| Zedernholzöl | 3,0 |
| Cashmeran^{®2} (1,2,3,5,6,7-Hexahydro-1,1,2,3,3-pentamethyl-⁴H-inden-4-on) | 5,5 |
| Iso E super^{®2} (2-Acetyl-1,2,3,4,5,6,7,8-octahydro-2,3,8,8-tetramethylnaphthalin) | 290,0 |
| Ambroxide krist.^{®4} (Dodecahydro-3a,6,6,9a-tetramethyl naphtho(2,1-b)furan) | 3,0 |
| Ambrettolide^{®4} (Oxacycloheptadec-10-en-2-on) | 7,0 |
| Ethylenbrassylat | 70,0 |
| Exaltenone^{®3} ((Z)-4-Cyclopentadecen-1-on) | 24,0 |
| Globalide^{®4} (Oxacyclohexadec-12-en-2-on) | 110,0 |
| Helvetolide^{®3} (2-[1-(3,3-Dimethyl-cyclohexyl)ethoxy]-2-methyl-1-propanolpropanoat | 2,0 |
| Dipropylenglycol | 143,0 |
| BHT Jonol | 1,0 |
| Summe | 880,0 |

| | |
|---|---|
| DPG = Dipropylenglycol ¹ Handelsname Givaudan AG, Schweiz; ² Handelsname International Flavors & Fragrances Inc., USA; ³ Handelsname Firmenich S.A., Schweiz; ⁴ Handelsname Symrise GmbH & Co. KG, Deutschland. | |

Der Zusatz von 20 Gewichtsteilen 6-Methyl-4-(2',2',3'-trimethyl-3'-cyclopenten-1'-yl)-2-cyclohexen-1-ol (VII) aus Beispiel 1.2 führt zu einer deutlich wahrnehmbaren Harmonisierung des gesamten Duftablaufs. Darüber hinaus verleiht die intensive, natürliche Sandelholzote der vorliegenden Komposition eine hervorragende Strahlung und Komplexität, gepaart mit einer gesteigerten Haftung. Hierbei setzt sich besonders der wertvolle Charakter von 6-Methyl-4-(2',2',3'-trimethyl-3'-cyclopenten-1'-yl)-2-cyclohexen-1-ol (VII) positiv durch.

### Beispiel 3: Shampoo

6-Methyl-4-(2',2',3'-trimethyl-3'-cyclopenten-1'-yl)-2-cyclohexen-1-ol (VII) (die erfindungsgemäße Verbindung) aus Beispiel 1.2 wurde als 50 Gew.%-ige Lösung in Diethylphthalat bereitgestellt, welche in einer Dosierung von 0,2 Gew.-% in eine Shampoo - Grundmasse der folgenden Rezeptur eingearbeitet wurde (Angaben in Gew.-%):

| | |
|---|---|
| Natriumlaurylethersulfat | 12% |
| (z.B. Texapon NSO, Fa. Cognis Deutschland GmbH) | |
| Cocamidopropylbetain | 2% |
| (z.B. Dehyton K, Fa. Cognis Deutschland GmbH) | |
| Natriumchlorid | 1,4% |
| Citronensäure | 1,3% |
| Phenoxyethanol, Methyl-, Ethyl-, Butyl-, | |
| und Propylparaben | 0,5% |
| Pfirsichriechstoffmischung umfasend gamma-Undecalacton | 0,5% |
| Wasser | 82,3% |

Der pH-Wert der Shampoo-Grundmasse lag bei etwa 6. Hieraus wurden 100 mL einer 20 Gew.%-igen wässrigen Shampoo-Lösung hergestellt. In dieser Shampoo-Lösung wurden 2 Haarsträhnchen gemeinsam für 2 Minuten gewaschen und anschließend 20 Sekunden unter fließendem handwarmen Wasser gespült. Eine Haarsträhne wurde nass in Aluminiumfolie eingepackt und die zweite Haarsträhne mit einem Fön getrocknet. Beide Haarsträhnen wurden von einem Panel von Geruchsexperten geruchlich beurteilt. Beide Haarsträhnen zeigten einen deutlichen sandelholzartigen Geruch, wobei der Gesamteindruck strahlend, abgerundet, natürlich und harmonisch empfunden wurde.

### Beispiel 4: Weichspüler

Die Parfümölkomposition aus Beispiel 2 (nach Zusatz der erfindungsgemäßen Verbindung (VII) aus Beispiel 1.2) wurde in einer Dosierung von 0,5 Gew.-% in eine Weichspüler-Grundmasse folgender Zusammensetzung eingearbeitet (Angaben in Gew.-%):

| | |
|---|---|
| Quarternäres Ammoniummethosulfat (Esterquat), ca. 90% | 5,5% |
| (z.B. Rewoquat WE 18, Fa. Witco Surfactants GmbH) | |
| Alkyldimethylbenzylammoniumchlorid, ca. 50% | 0,2% |
| (z.B. Preventol R50, Fa. Bayer AG) Farblösung, ca. 1%-ig | 0,3% |
| Wasser | 94,0% |

Der pH-Wert des Weichspüler-Grundmasse lag im Bereich 2 - 3. Zwei Stofflappen wurden mit 370 g einer 1%-igen wässrigen Weichspüler-Lösung in einer Linetest-Maschine im Weichspülprogramm 30 Minuten bei 20°C gespült. Die Lappen wurden ausgewrungen und anschließend 20 Sekunden geschleudert. Ein Lappen wurde nass eingeschweißt und einer zum Trocknen aufgehängt. Anschließend wurden beide Lappen durch ein Panel geruchlich beurteilt. Beide Lappen zeigten einen deutlichen sandelholzartigen Geruch, wobei der Gesamteindruck strahlend, abgerundet, natürlich und harmonisch empfunden wurde.

### Beispiel 5: Waschpulver

Die Parfümölkomposition aus Beispiel 2 (nach Zusatz der erfindungsgemäßen Verbindung (VII) aus Beispiel 1.2) wurde in einer Dosierung von 0,3 Gew.-% in eine Waschpulver-Grundmasse der folgenden Rezeptur eingearbeitet (alle Angaben in Gew.-%):

| | |
|---|---|
| Lineares Na-Alkylbenzolsulfonat | 8,8 % |
| Ethoxylierter Fettalkohol C12-18 (7 EO) | 4,7 % |
| Na-Seife | 3,2 % |
| Entschäumer | |
| DOW CORNING(R) 2-4248S | |
| POWDERED ANTIFOAM, | |
| Silikonöl auf Zeolith als Trägermaterial | 3,9 % |
| Zeolith 4A | 28,3 % |
| Na-Carbonat | 11,6 % |
| Na-Salz eines Copolymers aus Acryl- | |
| und Maleinsäure (Sokalan CP5) | 2,4 % |
| Na-Silicat | 3,0 % |
| Carboxymethylzellulose | 1,2 % |
| Dequest 2066 | 2,8 % |
| ([[(Phosphonomethyl)imino]bis[(ethylennitrilo)bis | |
| (methylen)]]tetrakis-phosphonsäure, Natriumsalz) | |
| Optischer Aufheller | 0,2 % |
| Na-Sulfat | 6,5 % |
| Protease | 0,4 % |
| Natriumperborattetrahydrat | 21,7 % |
| Riechstoffkomposition mit Rosengeruch | 0,3 % |
| TAED | 1,0 % |

Zwei Stofflappen wurden mit 370 g einer 1%-igen wässrigen Waschpulverlauge (der pH-Wert der Waschpulverlauge liegt deutlich im basischen Bereich) in einer Linetest-Maschine im Hauptwaschgang 45 Minuten bei 60°C gewaschen. Die Lappen wurden zunächst 5 Minuten mit kaltem Wasser gespült, ausgewrungen und anschließend 20 Sekunden geschleudert. Ein Lappen wurde nass eingeschweißt, und einer zum Trocknen aufgehängt. Anschließend wurden beide Lappen durch ein Panel geruchlich beurteilt. Beide Lappen zeigten einen deutlichen sandelholzartigen Geruch, wobei der Gesamteindruck strahlend, abgerundet, natürlich und harmonisch empfunden wurde.

### Beispiel 6: Deo-Stick

Die Parfümölkomposition aus Beispiel 2 (nach Zusatz der erfindungsgemäßen Verbindung (VII) aus Beispiel 1.2) wurde in einer Dosierung von 0,25 Gew.-% in eine Deo-Stick-Grundmasse der folgenden Rezeptur eingearbeitet:

| | Gew.-% |
|---|---|
| Natriumstearat | 8,0 |
| 1,2-Propylenglykol | 45,0 |
| 4-Methyl-4-phenyl-2-pentanol | 0,3 |
| 2-Hexyldecansäure | 0,5 |
| Polyethylenglycol(25)cetearylether | 3,0 |
| Ethanol | 20,0 |
| Wasser | Ad 100,0 |

Der Geruch des Deo-Sticks selbst sowie der in der Nähe der Achselhöhle wahrnehmbare Geruch nach Anwendung des Deo-Sticks zeigten einen deutlichen sandelholzartigen Aspekt, wobei der Gesamteindruck strahlend, abgerundet, natürlich und harmonisch empfunden wurde.

## Patentansprüche

1. Verbindung der Formel (VII).

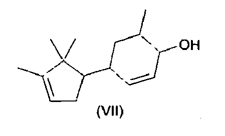

2. Verbindung nach Anspruch 1, wobei die Verbindung in Form
(a) eines reinen optisch aktiven Enantiomers,
(b) einer racemischen Mischung der Enantiomere eines, zweier, dreier oder aller diastereomeren Enantiomerenpaare oder
(c) einer optisch aktiven Mischung verschiedener Enantiomere vorliegt.

3. Riechstoff- oder Aromastoffkomposition, umfassend eine Verbindung nach Anspruch 1 oder 2 und wenigstens einen weiteren Riechstoff oder Aromastoff sowie vorzugsweise einen oder weitere übliche Bestandteile.

4. Parfümiertes oder aromatisiertes Produkt, umfassend eine Verbindung nach Anspruch 1 oder 2 oder eine Riechstoff- oder Aromastoffkomposition nach Anspruch 3.

5. Produkt nach Anspruch 4, ausgewählt aus der Gruppe bestehend aus Parfümölen und der Ernährung, der Mundpflege oder dem Genuss dienenden Zubereitungen.

6. Riechstoff- oder Aromastoffkomposition nach Anspruch 3 oder Produkt nach Anspruch 4 oder 5, umfassend eine als Sandelholzgeruchsnote olfaktorisch wahrnehmbare Menge einer Verbindung nach Anspruch 1 oder 2.

7. Produkt nach einem der Ansprüche 4 bis 6, umfassend einen Träger oder ein Substrat, der bzw. das in direktem Kontakt mit der Verbindung der Formel (VII) bzw. der Riechstoff- oder Aromastoffkomposition steht.

8. Verwendung einer Verbindung nach Anspruch 1 oder 2 als Riechstoff.

9. Verwendung einer Verbindung nach Anspruch 1 oder 2 zur Verbesserung der Fixierung einer Riechstoff- oder Aromastoffkomposition.

10. Verbindung der Formel (XII).

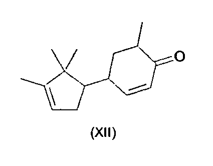

11. Verfahren zur Herstellung einer Verbindung der Formel (XII) nach Anspruch 10,
umfassend die Schritte:
- Bereitstellen von α-Methylencampholenaldehyd der Formel (XI) 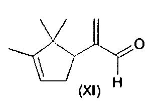 und
- Umsetzen des bereitgestellten α-Methylencampholenaldehyds der Formel (XI) mit 2-Methylacetessigsäureethylester in einer basenkatalysierten Cyclisierungs-Reaktion.

12. Verfahren zur Herstellung einer Verbindung nach einem der Ansprüche 1 oder 2, umfassend die Schritte:
- Bereitstellen oder Herstellen einer Verbindung der Formel (XII) nach Anspruch 10, und
- Reduzieren der Verbindung der Formel (XII), so dass eine Verbindung nach einem der Ansprüche 1 oder 2 gebildet wird.

13. Verfahren zum Erzeugen, Verstärken oder Modifizieren eines Sandelholzgeruchs in einer Mischung, umfassend folgende Schritte:
- Bereitstellen einer Verbindung nach einem der Ansprüche 1 oder 2,
- Bereitstellen einer Komposition sonstiger Bestandteile und
- Vermischen der Komposition sonstiger Bestandteile mit einer Menge der Verbindung nach einem der Ansprüche 1 oder 2, die ausreicht, um
(a) in der resultierenden Gesamtmischung einen Sandelholzgeruch zu erzeugen,
(b) einen vorhandenen Sandelholzgeruch in der Komposition sonstiger Bestandteile zu verstärken oder
(c) einen vorhandenen Sandelholzgeruch in der Komposition sonstiger Bestandteile zu modifizieren.

## Claims

1. Compound of the formula (VII)

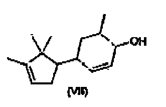

2. Compound according to claim 1, wherein the compound is in the form of
(a) a pure optically active enantiomer
(b) a racemic mixture of the enantiomers of one, two, three or all diastereomeric enantiomer pairs or
(c) an optically active mixture of various enantiomers.

3. Odoriferous substance or aroma substance composition, comprising a compound according to claim 1 or 2 and at least one further odoriferous substance or aroma substance and preferably one or further conventional constituents.

4. Perfumed or aromatized product, comprising a compound according to claim 1 or 2 or an odoriferous substance or aroma substance composition according to claim 3.

5. Product according to claim 4, chosen from the group consisting of perfume oils and formulations serving for nutrition, oral care or pleasure.

6. Odoriferous substance or aroma substance composition according to claim 3 or product according to claim 4 or 5, comprising an amount of a compound according to claim 1 or 2 which is olfactorily perceptible as a sandalwood olfactory note.

7. Product according to one of claims 4 to 6, comprising a carrier or a substrate which is in direct contact with the compound of the formula (VII) or the odoriferous substance or aroma substance composition.

8. Use of a compound according to claim 1 or 2 as an odoriferous substance.

9. Use of a compound according to claim 1 or 2 for improving the fixing of an odoriferous substance or aroma substance composition.

10. Compound of the formula (XII)

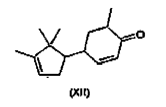

11. Process for the preparation of a compound of the formula (XII) according to claim 10,
comprising the steps:
- provision of α-methylenecampholenealdehyde of the formula (XI) 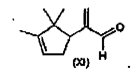 and
- reaction of the α-methylenecampholenealdehyde of the formula (XI) provided with 2-methylacetoacetic acid ethyl ester in a base-catalysed cyclization reaction.

12. Process for the preparation of a compound according to one of claims 1 or 2, comprising the steps:
- provision or preparation of a compound of the formula (XII) according to claim 10 and
- reduction of the compound of the formula (XII), so that a compound according to one of claims 1 or 2 is formed.

13. Method for generating, intensifying or modifying a sandalwood smell in a mixture, comprising the following steps:
- provision of a compound according to one of claims 1 or 2,
- provision of a composition of other constituents and
- mixing of the composition of other constituents with an amount of the compound according to one of claims 1 or 2 which is sufficient to
(a) generate a sandalwood smell in the resulting total mixture,
(b) intensify an existing sandalwood smell in the composition of other constituents or
(c) modify an existing sandalwood smell in the composition of other constituents.

## Revendications

1. Composé de la formule (VII)

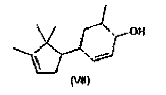

2. Composé selon la revendication 1, le composé se présentant sous la forme
(a) d'un énantiomère pur optiquement actif
(b) d'un mélange racémique des énantiomères d'une, de deux, de trois ou de toutes les paires d'énantiomères diastéréisomères.
(c) d'un mélange optiquement actif de différents énantiomères.

3. Composition de parfum ou d'aromatisant comportant un composé selon la revendication 1 ou 2 et au moins un autre parfum ou aromatisant, ainsi que de préférence un ou plusieurs autres composants usuels.

4. Produit parfumé ou aromatisé, comportant un composé selon la revendication 1 ou 2 ou une composition de parfum ou d'aromatisant selon la revendication 3.

5. Produit selon la revendication 4, choisi parmi le groupe consistant en les huiles de parfum et des préparations servant à l'alimentation, aux soins de la bouche ou au plaisir des sens.

6. Composition de parfum ou d'aromatisant selon la revendication 3 ou produit selon la revendication 4 ou 5, comprenant une quantité d'un composé selon la revendication 1 ou 2 perceptible par olfaction comme note de bois de santal.

7. Produit selon l'une quelconque des revendications 4 à 6, comprenant un porteur ou un substrat qui se trouve en contact direct avec le composé de la formule (VII), respectivement la composition de parfum ou d'aromatisant.

8. Utilisation d'un composé selon la revendication 1 ou 2 comme parfum.

9. Utilisation d'un composé selon la revendication 1 ou 2 pour l'amélioration de la fixation d'une composition de parfum ou d'aromatisant.

10. Composé de la formule (XII).

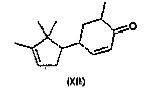

11. Procédé de préparation d'un composé de la formule (XII) selon la revendication 10,
comportant les étapes :
- mise à disposition de l'α-méthylènecampholène aldéhyde de la formule (XI) 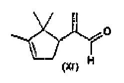 et
- réaction de l'α-méthylènecampholène aldéhyde de la formule (XI) mis à disposition avec le 2-méthylacétylacétate d'éthyle dans une réaction de cyclisation catalysée par base.

12. Procédé de préparation d'un composé selon l'une quelconque des revendications 1 ou 2, comprenant les étapes :
- mise à disposition ou préparation d'un composé de la formule (XII) selon la revendication 10 et
- réduction du composé de la formule (XII), de telle sorte qu'un composé selon l'une quelconque des revendications 1 ou 2 soit formé.

13. Procédé pour produire, renforcer ou modifier un parfum de bois de santal dans un mélange, comportant les étapes suivantes :
- mise à disposition d'un composé selon l'une quelconque des revendications 1 ou 2,
- mise à disposition d'une composition d'autres constituants et
- mélangeage de la composition d'autres constituants avec une quantité du composé selon l'une quelconque des revendications 1 ou 2, qui suffit pour
(a) produire dans le mélange global résultant un parfum de bois de santal,
(b) renforcer dans la composition d'autres constituants un parfum de bois de santal existant ou
(c) modifier dans la composition d'autres constituants un parfum de bois de santal existant.
